# EUROPEAN PATENT SPECIFICATION

(11) **EP 3 572 110 B1**
(45) Date of publication and mention of the grant of the patent: **14.09.2022**
(21) Application number: 18740998.2
(22) Date of filing: 12.01.2018
(51) Int. Cl.: A61M 16/00, A61M 16/06, A61M 16/10

(54) **OXYGEN CONCENTRATOR**
SAUERSTOFFKONZENTRATOR
CONCENTRATEUR D'OXYGÈNE

(30) Priority: 20.01.2017 JP 2017008148
(43) Date of publication of application: 27.11.2019
(73) Proprietor: Daikin Industries, Ltd., Osaka-shi, Osaka 530-8323 (JP)
(72) Inventor: KONDOU, Keita, Osaka-Shi, Osaka 530-8323 (JP); HIRANO, Tomoya, Osaka-Shi, Osaka 530-8323 (JP); HIEI, Takehiko, Osaka-Shi, Osaka 530-8323 (JP); IWAKAME, Makoto, Osaka-Shi, Osaka 530-8323 (JP)
(74) Representative: Goddar, Heinz J.
(86) International application number: PCT/JP2018/000552
(87) International publication number: WO 2018/135377

(56) References cited:
- WO-A1-2011/019091
- WO-A1-2014/083595
- JP-A- H11 314 903
- JP-A- 2004 242 906
- JP-A- 2007 032 088
- JP-A- 2008 119 288
- JP-A- 2008 511 399
- JP-A- 2009 050 347
- JP-A- 2016 501 589
- US-A- 4 744 356
- US-A1- 2002 038 656
- US-A1- 2006 048 781
- US-A1- 2006 219 245
- US-A1- 2011 232 642

## Description

### [Technical Field]

The present teaching relates to an oxygen concentrator configured to supply oxygen to a patient.

### [Background Art]

A known oxygen concentrator is arranged to switch on/off oxygen supply in accordance with a timing of patient's respiration. In such an oxygen concentrator, a timing at which the patient's respiration is switched from exhalation to inhalation is detected and oxygen supply is switched on (see Patent Literature 1).

### [Citation List]

### [Patent Literature]

[Patent Literature 1] Japanese Unexamined Patent Publication No. 2002-085567 US 2006/048781 A1, US 2002/038656 A1, US 2011/232642 A1, US 2006/219245 A1 disclose systems and methods for providing oxygen therapy.

### [Summary]

### [Technical Problem]

In the oxygen concentrator recited in Patent Literature 1, a negative pressure at the start of the inhalation of the patient is detected by a pressure sensor, and oxygen supply is controlled. In this way, this oxygen concentrator merely detects the start of inhalation of the patient, and does not detect the waveform of patient's respiration. If an oxygen concentrator is capable of detecting a respiration waveform, it is considered that the state of health of a patient is graspable. Such an oxygen concentrator, however, did not exist.

The present teaching has been done to solve the problem above, and an object of the present teaching is to provide an oxygen concentrator which is configured to detect an waveform of patient's respiration and is able to detect the state of health of the patient.

### [Solution to Problem]

The present invention provides an oxygen concentrator according to claim 1.

According to the present teaching, a waveform of patient's respiration is directly detected by the respiration waveform sensor connected to at least one of the passage and the cannula, the passage connecting the oxygen generation unit to the oxygen discharge unit, the cannula attached to the oxygen discharge unit. The respiration waveform detected by the respiration waveform sensor is stored in the respiration waveform storage unit. This makes it possible to grasp the state of health of the patient.

The oxygen concentrator of the present teaching may further include: a flow amount adjustment unit provided on the passage; and a control unit configured to control the flow amount adjustment unit based on the respiration waveform detected by the respiration waveform sensor.

This arrangement makes it possible to supply oxygen to the patient, with a suitable flow amount of oxygen based on the waveform of patient's respiration.

The oxygen concentrator of the present teaching is arranged such that the control unit controls the flow amount adjustment unit so that: the oxygen is discharged from the respiration waveform when the respiration waveform sensor detects the respiration waveform; and the oxygen is not discharged from the oxygen discharge unit when the respiration waveform sensor does not detect the respiration waveform.

When the respiration waveform sensor detects a respiration waveform, it is considered that the patient wears the cannula. Meanwhile, when the respiration waveform sensor does not detect a respiration waveform, it is considered that the patient does not wear the cannula. According to the arrangement above, it is possible to supply necessary oxygen to the patient and at the same time prevent unnecessary oxygen supply, by supplying oxygen to the patient when the respiration waveform sensor detects a respiration waveform and stopping oxygen supply to the patient when the respiration waveform sensor does not detect a respiration waveform.

The oxygen concentrator of the present teaching is arranged such that the control unit controls the flow amount adjustment unit so as not to discharge the oxygen from the oxygen discharge unit for a predetermined time during an exhalation time, based on the respiration waveform detected by the respiration waveform sensor.

According to this arrangement, oxygen supply is stopped for a predetermined time during the exhalation time. This reduces an amount of supplied oxygen, and hence the oxygen tank can be used for a longer time. To put it differently, stop of oxygen supply is triggered by the detection of exhalation by a patient. When exhalation of a patient is not detected (e.g., when the respiration waveform sensor does not detect a waveform of patient's respiration), oxygen supply is continued. This allows the patent to receive oxygen even during sleep.

The oxygen concentrator of the present teaching may further include: a flow amount storage unit configured to store a plurality of flow amounts corresponding to a plurality of states of a patient; a state detection unit configured to detect a state of the patient based on the respiration waveform detected by the respiration waveform sensor; and a state flow amount determination unit configured to detect a flow amount, corresponding to the state of the patient detected by the state detection unit, in the plurality of flow amounts stored un the flow amount storage unit wherein the control unit controls the flow amount adjustment unit so that the oxygen with the flow amount determined by the flow amount determination unit is discharged from the oxygen discharge unit.

This arrangement makes it possible to supply a suitable flow amount of oxygen to the patient.

The oxygen concentrator of the present teaching may further include: a fire waveform storage unit configured to store a waveform of a fire; and a fire detection unit configured to detect that the respiration waveform detected by the respiration waveform sensor is identical with the waveform of the fire stored in the fire waveform storage unit, the control unit controls the flow amount adjustment unit so that the oxygen is not discharged from the oxygen discharge unit, when the fire detection unit detects that the respiration waveform detected by the respiration waveform sensor is identical with the waveform of the fire stored in the fire waveform storage unit.

This arrangement prevents the fire from spreading.

The oxygen concentrator of the present teaching may further include: an input unit to which length of the cannula is input; a correction amount storage unit configured to store a correction amount corresponding to the length of the cannula; a correction amount determination unit configured to determine a correction amount corresponding to the length of the cannula input to the input unit, with reference to the correction amount stored in the correction amount storage unit; and a correction unit configured to correct the amplitude of the respiration waveform detected by the respiration waveform sensor, based on the correction amount determined by the correction amount determination unit.

With this arrangement, the respiration waveform sensor is able to certainly detect the respiration waveform irrespective of the length of the cannula. The length of the cannula the total of the length of the cannula itself and the length of an extension tube when the extension tube is connected to the cannula.

The oxygen concentrator of the present teaching may be arranged such that the cannula includes a contact portion which is in contact with an ear of the patient when the cannula is attached to a nose of the patient, the respiration waveform sensor is configured to detect heartbeat of the patient through the contact portion, in addition to the respiration waveform of the patient, and the respiration waveform and the heartbeat detected by the respiration waveform sensor are stored in the respiration waveform storage unit.

According to the arrangement above, in addition to a waveform of patient's respiration, heartbeat detected through the contact portion is stored in the respiration waveform storage unit. This makes it possible to grasp the state of health of the patient.

### [Advantageous Effects]

According to the present teaching, a waveform of patient's respiration is directly detected by the respiration waveform sensor connected to at least one of the passage and the cannula, the passage connecting the oxygen generation unit to the oxygen discharge unit, and the cannula attached to the oxygen discharge unit. The respiration waveform detected by the respiration waveform sensor is stored in the respiration waveform storage unit. This makes it possible to grasp the state of health of the patient.

### [Brief Description of Drawings]

FIG. 1 is a block diagram of an oxygen concentrator of First Embodiment of the present teaching.
FIG. 2 is a graph showing an example of a waveform of patient's respiration.
FIG. 3 is a flow chart related to First Embodiment of the present teaching.
FIG. 4 is a block diagram of an oxygen concentrator of Second Embodiment of the present teaching.
FIG. 5 is a flow chart related to Second Embodiment of the present teaching.
FIG. 6 is a block diagram of an oxygen concentrator of Third Embodiment of the present teaching.
FIG. 7 is a graph showing an example of a waveform of fire.
FIG. 8 is a flow chart related to Third Embodiment of the present teaching.
FIG. 9 is a block diagram of an oxygen concentrator of Fourth Embodiment of the present teaching.
FIG. 10 is a flow chart related to Fourth Embodiment of the present teaching.
FIG. 11 is a block diagram of an oxygen concentrator of Fifth Embodiment of the present teaching.
FIG. 12 is a graph showing an example of patient's heartbeat.
FIG. 13 is a table showing an exhalation time and an inhalation time when the respiratory frequency per minute is 10 times to 40 times.
FIG. 14 is a graph showing a respiration waveform detected by a respiration waveform sensor.
FIG. 15 is a flow chart related to Sixth Embodiment of the present teaching. The invention is defined by the sixth embodiment, presented in figures 14 and 15. The rest of the figures and embodiments do not form part of the present invention.

### [Description of Embodiments]

The following describes an embodiment of the present teaching with reference to attached drawings.

### <First Embodiment>

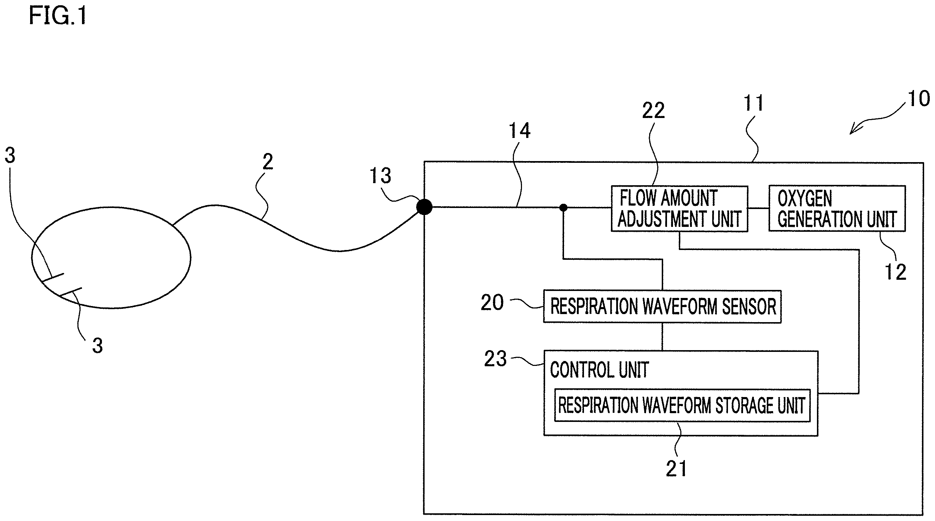

As shown in FIG. 1, an oxygen concentrator 10 of First Embodiment is connected to a cannula 2 which is used for allowing a patient receiving oxygen inhalation therapy to inhale oxygen through the nose, and the oxygen concentrator 10 supplies oxygen to the cannula 2. The oxygen concentrator 10 may be connected to an instrument for discharging oxygen, which is not a cannula.

The oxygen concentrator 10 includes a main body 11, an oxygen generation unit 12 provided in the main body 11, and an oxygen discharge unit 13 to which the cannula 2 is attached. The oxygen generation unit 12 is connected to the oxygen discharge unit 13 by a passage 14. The oxygen generation unit 12 is therefore connected to the cannula 2 via the passage 14 and the oxygen discharge unit 13. The oxygen generation unit 12 is configured to generate concentrated oxygen gas. The oxygen discharge unit 13 is configured to discharge oxygen generated by the oxygen generation unit 12 to the cannula 2. At a leading end of the cannula 2, paired attaching parts 3 are provided to be attached the nose of the patient.

On the passage 14, a flow amount adjustment unit 22 configured to adjust an oxygen flow amount supplied to the patient is provided between the oxygen generation unit 12 and the oxygen discharge unit 13. The flow amount adjustment unit 22 is connected to a control unit 23 which is configured to control the flow amount adjustment unit 22.

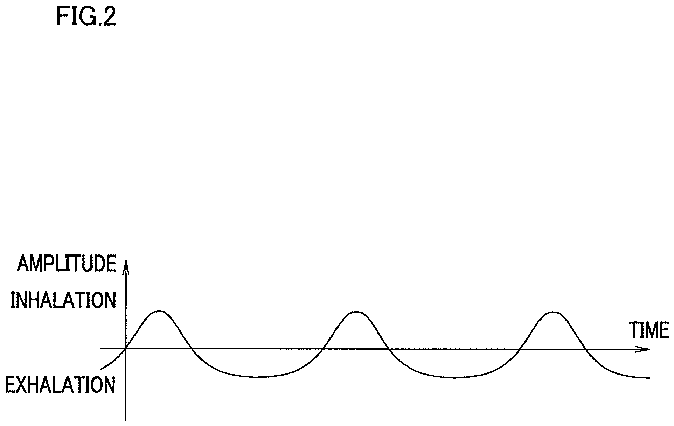

On the passage 14, a respiration waveform sensor 20 configured to detect a waveform of patient's respiration is provided between the flow amount adjustment unit 22 and the oxygen discharge unit 13. The respiration waveform sensor 20 is a capacitor microphone using an electret element which is semi-permanently charged. The capacitor microphone is able to detect a dynamic pressure change at low frequencies such as 0.5Hz, and is suitable for sound pressure measurement of 1Pa or lower. A respiration waveform detected by the respiration waveform sensor 20 is, as shown in FIG. 2, for example, represented by a graph in which exhalation and inhalation are alternately and periodically repeated. The respiration waveform sensor 20 is connected to a respiration waveform storage unit 21 which stores a respiration waveform detected by the respiration waveform sensor 20. The respiration waveform storage unit 21 is housed in the control unit 23.

When the oxygen concentrator 10 is driven, the oxygen generation unit 12 generates concentrated oxygen gas by means of adsorbent such as zeolite, which adsorbs nitrogen under a high pressure and desorbs the adsorbed nitrogen under a low pressure. In other words, the oxygen generation unit 12 is configured to generate concentrated oxygen gas by compressing air taken into the main body 11 from the outside of the main body 11 and adsorbing nitrogen in the compressed air. The nitrogen desorbed from the adsorbent under a low pressure is discharged to the outside. Meanwhile, the concentrated oxygen gas generated by the oxygen generation unit 12 reaches the oxygen discharge unit 13 via the passage 14, is discharged from the oxygen discharge unit 13 to the cannula 2, and is eventually supplied to the patient.

In the oxygen concentrator 10 of First Embodiment, the control unit 23 controls the flow amount adjustment unit 22 so that: oxygen is discharged from the oxygen discharge unit 13 when the respiration waveform sensor 20 detects a respiration waveform; whereas no oxygen is discharged from the oxygen discharge unit 13 when the respiration waveform sensor 20 does not detect a respiration waveform.

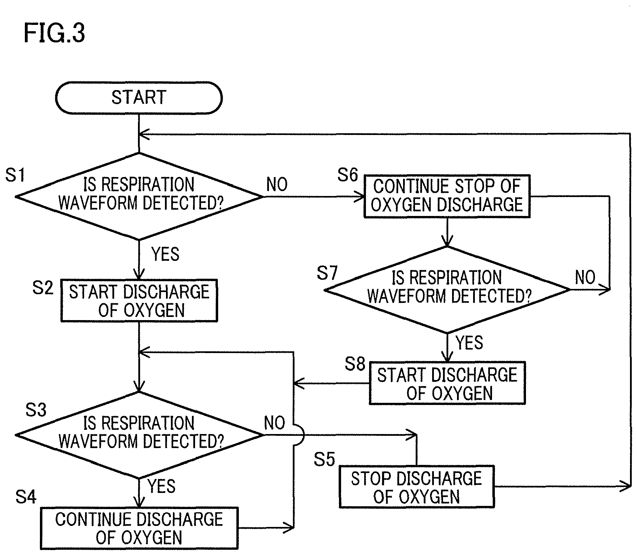

To be more specific, to begin with, the control unit 23 determines whether the respiration waveform sensor 20 detects a respiration waveform, as shown in FIG. 3 (step S1). When the respiration waveform sensor 20 detects a respiration waveform (Yes in S1), the control unit 23 controls the flow amount adjustment unit 22 to start the discharge of oxygen (step S2) . After the step S2, when the respiration waveform sensor 20 continues the detection of the respiration waveform and the respiration waveform sensor 20 detects the respiration waveform (Yes in S3), the control unit 23 controls the flow amount adjustment unit 22 to continue the discharge of oxygen (step S4) . After the step S4, the control unit 23 returns the process to the step S3. When the respiration waveform sensor 20 does not detect a respiration waveform (No in S3), the control unit 23 controls the flow amount adjustment unit 22 to stop the discharge of oxygen (step S5) . After the step S5, the control unit 23 returns the process to the step S1.

When the respiration waveform sensor 20 does not detect a respiration waveform (No in S1), the control unit 23 controls the flow amount adjustment unit 22 to keep the discharge of oxygen stopped (step S6). After the step S6, when the respiration waveform sensor 20 continues the detection of a respiration waveform and the respiration waveform sensor 20 detects a respiration waveform (Yes in S7), the control unit 23 controls the flow amount adjustment unit 22 to start the discharge of oxygen (step S8) . After the step S8, the control unit 23 proceeds to the step S3. When the respiration waveform sensor 20 does not detect a respiration waveform (No in S7), the control unit 23 returns the process to the step S6.

In the oxygen concentrator 10 of First Embodiment, the respiration waveform sensor 20 connected to the passage 14 which connects the oxygen generation unit 12 to the oxygen discharge unit 13 is able to directly detect a waveform of patient's respiration. The respiration waveform detected by the respiration waveform sensor 20 is stored in the respiration waveform storage unit 21. This makes it possible to grasp the state of health of the patient.

In the oxygen concentrator 10 of First Embodiment, the control unit 23 controls the flow amount adjustment unit 22 provided on the passage 14 connecting the oxygen generation unit 12 to the oxygen discharge unit 13, based on a respiration waveform detected by the respiration waveform sensor 20. This makes it possible to supply oxygen to the patient, with a suitable flow amount of oxygen based on the waveform of patient's respiration.

In the oxygen concentrator 10 of First Embodiment, the control unit 23 controls the flow amount adjustment unit 22 so that: oxygen is discharged from the oxygen discharge unit 13 when the respiration waveform sensor 20 detects a respiration waveform; whereas no oxygen is discharged from the oxygen discharge unit 13 when the respiration waveform sensor 20 does not detect a respiration waveform. When the respiration waveform sensor 20 detects a respiration waveform, it is considered that the patient wears the cannula 2. Meanwhile, when the respiration waveform sensor 20 does not detect a respiration waveform, it is considered that the patient does not wear the cannula 2. According to the arrangement above, it is possible to supply necessary oxygen to the patient and at the same time prevent unnecessary oxygen supply, by supplying oxygen to the patient when the respiration waveform sensor 20 detects a respiration waveform and stopping oxygen supply to the patient when the respiration waveform sensor 20 does not detect a respiration waveform.

### <Second Embodiment>

An oxygen concentrator 10 of Second Embodiment is configured to supply oxygen to a patient, with a flow amount suitable for the state of the patient. In regard to elements in Second Embodiment, elements identical with those in First Embodiment are denoted by the same reference symbols and are not explained again.

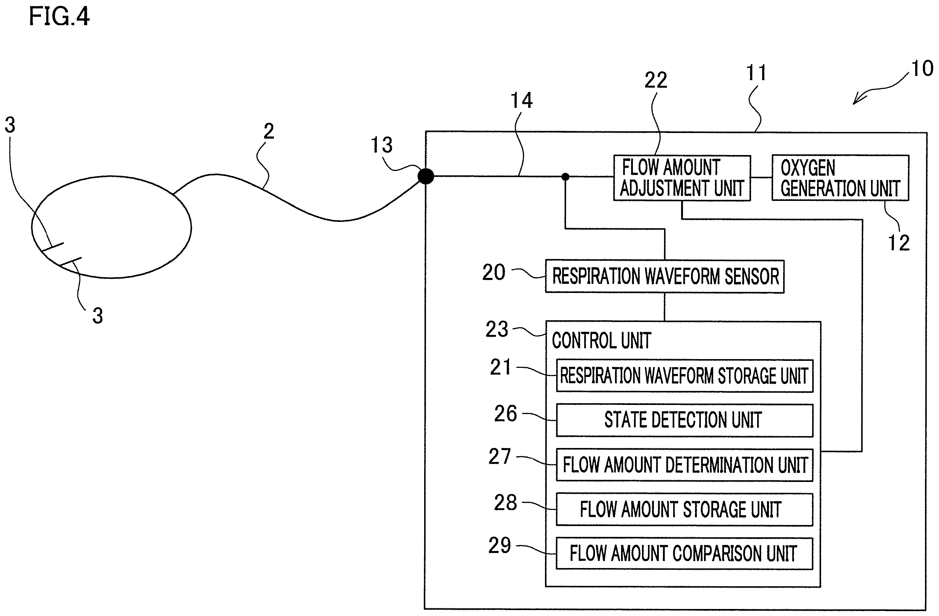

As shown in FIG. 4, a respiration waveform sensor 20 is connected to a passage 14 connecting an oxygen generation unit 12 to an oxygen discharge unit 13. A control unit 23 is connected to the respiration waveform sensor 20 and a flow amount adjustment unit 22. In the control unit 23, a respiration waveform storage unit 21, a state detection unit 26, a flow amount determination unit 27, a flow amount storage unit 28, and a flow amount comparison unit 29 are housed.

The flow amount storage unit 28 is configured to store a flow amount of supplied oxygen in accordance with the state of a patient. The state of the patient is, for example, a rest state, an exercising state, or a sleep state. The flow amount of supplied oxygen in accordance with the state of the patient is, to be more specific, 2 liters in the rest state, 2.5 liters in the exercising state, or 1.5 liters in the sleep state. These flow amounts of supplied oxygen are prescribed by a physician in advance, and are stored in the flow amount storage unit 28.

The state detection unit 26 is configured to detect the above-described state of the patient based on a respiration waveform detected by the respiration waveform sensor 20 (to be more specific, the magnitude of the variation range of the respiratory frequency). In order to detect the state of the patient, the state detection unit 26 calculates the respiratory rate of the patient from the respiration waveform detected by the respiration waveform sensor 20, and figures out a variation coefficient per predetermined time. The variation coefficient is a coefficient calculated by dividing a standard deviation of a respiratory rate by an average respiratory rate per unit time, and is one of three stages which are large, middle, and small. For example, when the respiratory rate is equal to or smaller than 20bpm and the variation coefficient is "middle", the state of the patient is the rest state. When the respiratory rate is larger than 20bpm or the variation coefficient is "large", the state of the patient is the exercising state. When the respiratory rate is equal to or smaller than 20bpm and the variation coefficient is "small", the state of the patient is the sleep state. The state detection unit 26 performs the detection of the state of the patient (rest state, exercising state, or sleep state) when each state is maintained for a predetermined time.

The flow amount determination unit 27 is configured to determine the flow amount (which is one of 2 liters, 2.5 liters, and 1.5 liters) corresponding to the state of the patient detected by the state detection unit 26. To the passage 14, a flow amount adjustment unit 22 is connected to adjust the oxygen flow amount supplied to the patient. The flow amount adjustment unit 22 and the flow amount determination unit 27 are connected to a control unit 23 which is configured to control the flow amount adjustment unit 22.

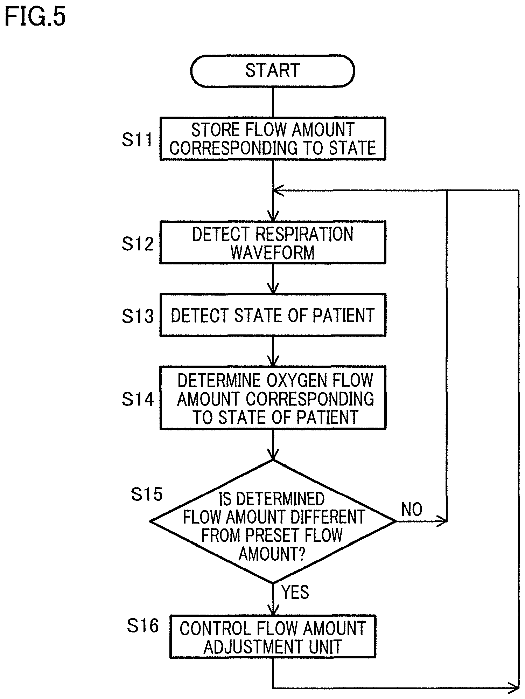

The following will describe the steps of using the oxygen concentrator 10 of Second Embodiment with reference to FIG. 5.

To begin with, a physician prescribes an oxygen flow amount corresponding to the state of a patient (rest state, exercising state, or sleep state). When the oxygen concentrator 10 is driven and the prescribed oxygen flow amount is input by the patient through an input unit (not illustrated) of the oxygen concentrator 10, the flow amount storage unit 28 stores the input flow amount (step S11). After the step S11, the respiration waveform sensor 20 detects an waveform of patient's respiration (step S12) . After the step S12, the state detection unit 26 detects that the state of the patient is the rest state, the exercising state, or the sleep state, based on the respiration waveform detected by the respiration waveform sensor 20 (step S13). After the step S13, in accordance with the state of the patient detected by the state detection unit 26, the flow amount determination unit 27 determines that the oxygen flow amount is 2 liters, 2.5 liters, or 1.5 liters (step S14). After the step S14, the flow amount comparison unit 29 determines whether the flow amount determined by the flow amount determination unit 27 is different from a preset flow amount (step S15). The preset flow amount is an oxygen flow amount supplied to the patient when the respiration waveform is detected in the step S12. When the flow amount determined by the flow amount determination unit 27 is not different from the preset flow amount (No in the step S15), the control unit 23 returns the process to the step S12. When the flow amount determined by the flow amount determination unit 27 is different from the preset flow amount (Yes in the step S15), the control unit 23 controls the flow amount adjustment unit 22 so that the oxygen is discharged from the oxygen discharge unit 13 with the flow amount determined by the flow amount determination unit 27 (step S16). After the step S16, the control unit 23 returns the process to the step S12.

In the oxygen concentrator 10 of Second Embodiment, the state detection unit 26 detects the state of the patient based on the respiration waveform. Therefore a flow amount suitable for the state is determined by the flow amount determination unit 27. This makes it possible to supply a suitable flow amount of oxygen to the patient.

### <Third Embodiment>

An oxygen concentrator 10 of Third Embodiment is configured to stop supply of oxygen to a patient when a fire occurs. In regard to elements in Third Embodiment, elements identical with those in First Embodiment are denoted by the same reference symbols and are not explained again.

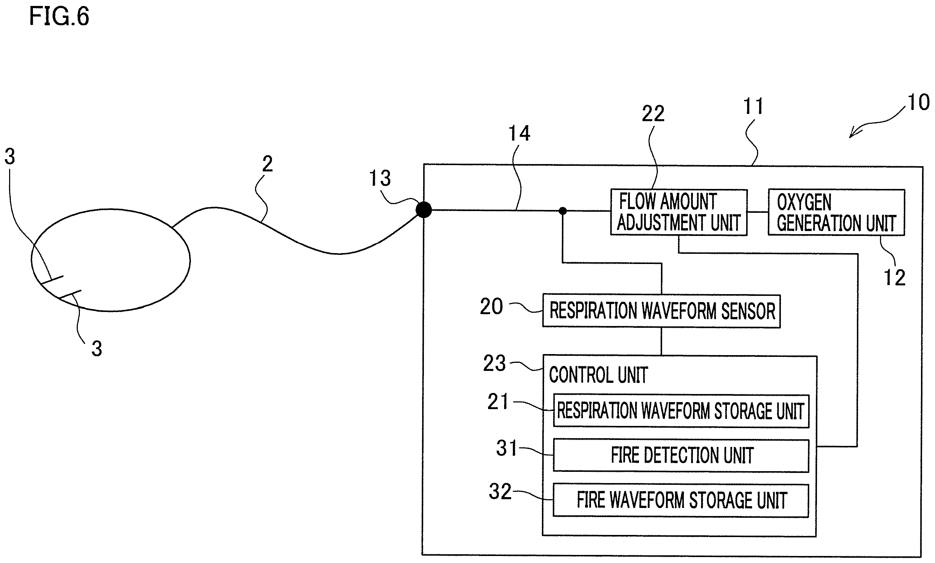

As shown in FIG. 6, a respiration waveform sensor 20 is connected to a passage 14 connecting an oxygen generation unit 12 to an oxygen discharge unit 13. A control unit 23 is connected to the respiration waveform sensor 20 and a flow amount adjustment unit 22. In the control unit 23, a respiration waveform storage unit 21, a fire detection unit 31, and a fire waveform storage unit 32 are housed.

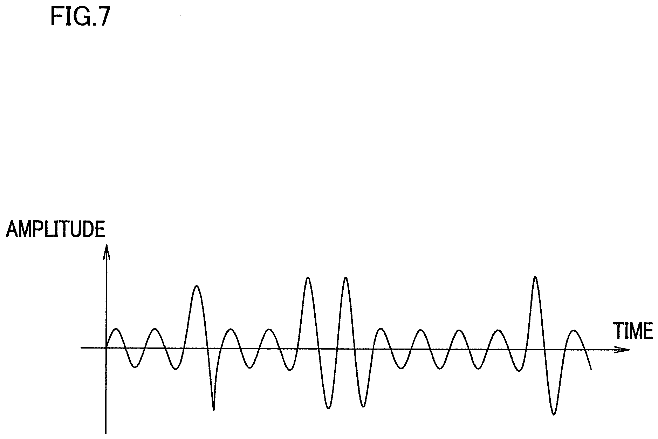

The fire waveform storage unit 32 is configured to store a waveform of fire. As shown in FIG. 7, being different from a respiration waveform, a waveform of fire is arranged such that parts with large amplitudes suddenly appear in standing waves with small amplitudes. The fire detection unit 31 is configured to detect that a waveform detected by the respiration waveform sensor 20 is identical with a waveform of fire stored in the fire waveform storage unit 32.

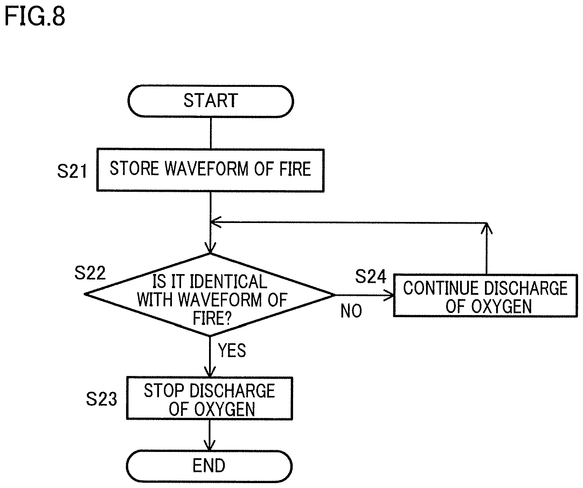

The following will describe a flow of detection of a fire by the oxygen concentrator 10 of Third Embodiment with reference to FIG. 8.

To begin with, when a waveform of a fire is input through an input unit (not illustrated) of the oxygen concentrator 10, the fire waveform storage unit 32 stores the waveform (step S21) . After the step S21, after oxygen supply to a patient starts, the fire detection unit 31 determines whether a waveform detected by the respiration waveform sensor 20 is identical with the waveform of fire stored in the fire waveform storage unit 32 (step S22). When the waveform detected by the respiration waveform sensor 20 is identical with the waveform of fire stored in the fire waveform storage unit 32 (Yes in S22), it is determined that a fire has occurred. In this case, the control unit 23 controls the flow amount adjustment unit 22 not to discharge oxygen from the oxygen discharge unit 13 (step S23). The process is terminated after the step S23. When a waveform detected by the respiration waveform sensor 20 is not identical with the waveform of fire stored in the fire waveform storage unit 32 (No in the step S22), the control unit 23 keeps oxygen discharged from the oxygen discharge unit 13 (step S24). After the step S24, the control unit 23 returns the process to the step S22.

In the oxygen concentrator 10 of Third Embodiment, the control unit 23 controls the flow amount adjustment unit 22 so that oxygen is not discharged from the oxygen discharge unit 13 when the fire detection unit 31 determines that a waveform detected by the respiration waveform sensor 20 is identical with the waveform of fire stored in the fire waveform storage unit 32. This prevents the fire from spreading.

### <Fourth Embodiment>

An oxygen concentrator 10 of Fourth Embodiment is configured to correct the amplitude of a respiration waveform which varies depending on the length of a cannula 2. The cannula 2 in the present embodiment is a cannula to which an extension tube (not illustrated) is connected. On this account, the length of the cannula 2 is the total of the length of the cannula and the length of the extension tube. In regard to elements in Fourth Embodiment, elements identical with those in First Embodiment are denoted by the same reference symbols and are not explained again.

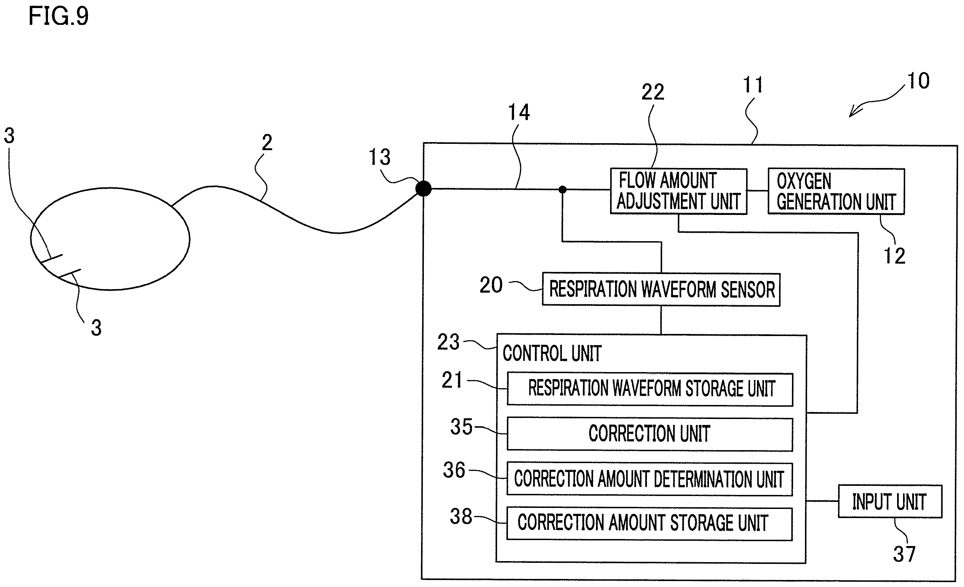

As shown in FIG. 9, a respiration waveform sensor 20 is connected to a passage 14 connecting an oxygen generation unit 12 to an oxygen discharge unit 13. A control unit 23 is connected to the respiration waveform sensor 20 and a flow amount adjustment unit 22. In the control unit 23, a respiration waveform storage unit 21, a correction unit 35, a correction amount determination unit 36, and a correction amount storage unit 38 are housed. The control unit 23 is connected to an input unit 37 to which the length of the cannula 2 is input.

The correction amount storage unit 38 stores a correction amount corresponding to the length of the cannula 2. The correction amount determination unit 36 is configured to determine a correction amount corresponding to the length of the cannula 2 input to the input unit 37, with reference to the correction amount stored in the correction amount storage unit 38. The correction unit 35 is configured to correct the amplitude of a respiration waveform detected by the respiration waveform sensor 20, based on the correction amount determined by the correction amount determination unit 36.

When the respiration waveform sensor 20 detects a waveform of patient's respiration in oxygen inhalation, respiratory sound vibration typically attenuates as the length of the cannula 2 increases. On this account, the amplitude of a respiration waveform detected by the respiration waveform sensor 20 when the cannula 2 is long is smaller than when the cannula 2 is short. In the present embodiment, the amplitude of a respiration waveform detected by the respiration waveform sensor 20 is corrected by the correction unit 35. This causes the amplitude of a respiration waveform to be constant irrespective of the length of the cannula 2. The correction amount corresponding to the length of the cannula 2 is set as described below, with the assumption that the correction amount is 0 (reference value) when the length of the cannula 2 is 1 meter and the amplitude of the respiration waveform is 1. Therefore the correction amount is 0.1 when the length of the cannula 2 is 8 meters and the amplitude of the respiration waveform is 0.9, and the correction amount is 0.2 when the length of the cannula 2 is 15 meters and the amplitude of the respiration waveform is 0.8.

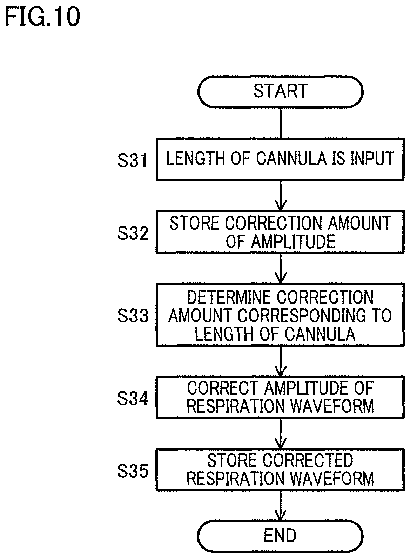

The following will describe a flow of correction of the amplitude of a respiration waveform by the oxygen concentrator 10 of Fourth Embodiment with reference to FIG. 10.

To begin with, the length of the cannula 2 is input through the input unit 37 (step S31) . After the step S31, the correction amount storage unit 38 stores a correction amount of the amplitude of a waveform corresponding to the length of the cannula 2 (step S32) . After the step S32, the correction amount determination unit 36 determines a correction amount corresponding to the length of the cannula 2 input to the input unit 37, with reference to the correction amount stored in the correction amount storage unit 38 (step S33). After the step S33, the correction unit 35 corrects the amplitude of the respiration waveform detected by the respiration waveform sensor 20, based on the correction amount determined by the correction amount determination unit 36 (step S34) . After the step S34, the control unit 23 stores the corrected respiration waveform in the respiration waveform storage unit 21 (step S35) . The process is terminated after the step S35.

In the oxygen concentrator 10 of Fourth Embodiment, the correction amount determination unit 36 determines a correction amount corresponding to the length of the cannula 2 input to the input unit 37, with reference to the correction amount stored in the correction amount storage unit 38. The correction unit 35 then corrects the amplitude of the respiration waveform detected by the respiration waveform sensor 20, based on the correction amount determined by the correction amount determination unit 36. The respiration waveform sensor 20 is therefore able to certainly detect the respiration waveform irrespective of the length of the cannula 2.

### <Fifth Embodiment>

An oxygen concentrator 10 of Fifth Embodiment is configured to detect and store heartbeat in addition to a waveform of patient's respiration. In regard to elements in Fifth Embodiment, elements identical with those in First Embodiment are denoted by the same reference symbols and are not explained again.

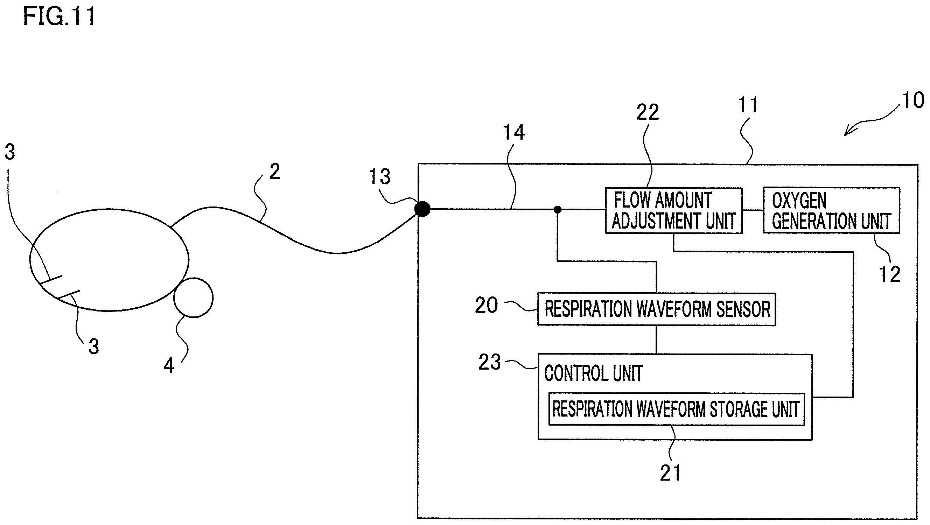

As shown in FIG. 11, a cannula 2 is provided with a contact portion 4 which is in contact with an ear of a patient when the cannula 2 is attached to the nose of the patient. As the contact portion 4 is hooked on the ear of the patient, the state of attachment of the attaching parts 3 to the nose is maintained. The block diagram of the oxygen concentrator 10 shown in FIG. 11 is identical with the block diagram of First Embodiment except the presence of the contact portion 4.

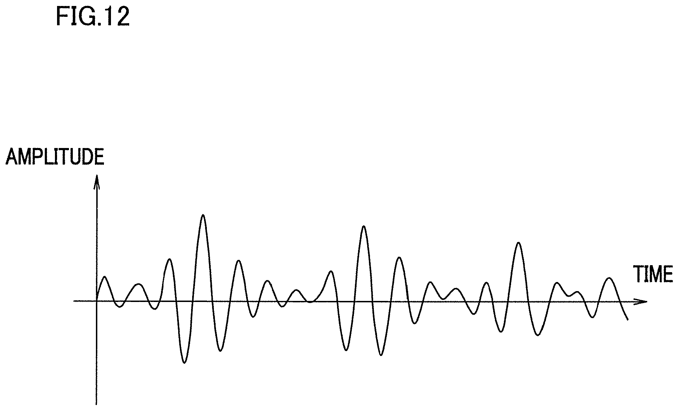

A respiration waveform sensor 20 is configured to detect the heartbeat of a patient through the contact portion 4, in addition to a waveform of patient's respiration. The respiration waveform and heartbeat detected by the respiration waveform sensor 20 are stored in the respiration waveform storage unit 21. As shown in FIG. 12, heartbeat is arranged such that waveforms with small amplitudes and waveforms with gradually increasing amplitudes are alternately repeated.

In the oxygen concentrator 10 of Fifth Embodiment, in addition to a waveform of patient's respiration, heartbeat detected through the contact portion 4 is stored in the respiration waveform storage unit 21. This makes it possible to grasp the state of health of the patient.

### <Sixth Embodiment>

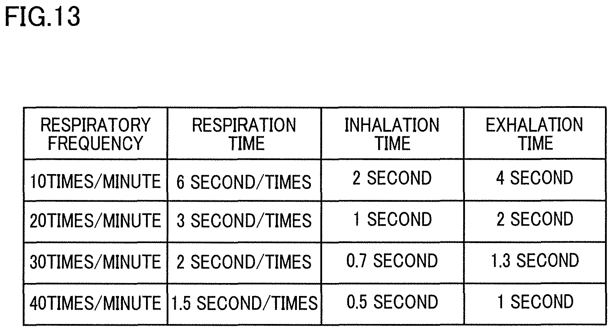

An oxygen concentrator 10 of Sixth Embodiment is configured to stop oxygen supply for a predetermined time during an exhalation time (i.e., control a flow amount adjustment unit 22 not to discharge oxygen from an oxygen discharge unit 13) . The ratio of an inhalation time to an exhalation time in human respiration is 1:2. The exhalation time is longer than the inhalation time. FIG. 13 shows the exhalation time and the inhalation time when the respiratory frequency per minute is 10 times, 20 times, 30 times, or 40 times. When the respiratory frequency per minute is 10 times, one respiration is performed in 6 seconds, and the inhalation time is 2 seconds whereas the exhalation time is 4 seconds in one respiration.

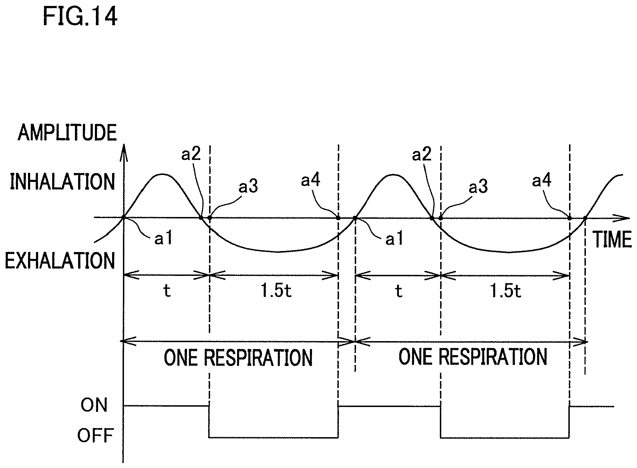

FIG. 14 shows a respiration waveform detected by a respiration waveform sensor 20. In FIG. 14, a patient starts inhalation at the leftmost point a1, and the inhalation is switched to exhalation at the point a2. The exhalation ends at the next point a1 and inhalation starts. To put it differently, the patient performs respiration once during the time from the point a1 to the next point a1. In FIG. 14, the point a3 indicates a point at which oxygen supply to the patient is stopped, and the point a4 indicates a point at which oxygen supply to the patient starts. The time from the detection of the inhalation of the patient at the point a1 by the respiration waveform sensor 20 to the stop of oxygen supply at the point a3 is referred to as t seconds, and the time from the stop of oxygen supply at the point a3 to the start of oxygen supply at the point a4 is referred as 1.5t seconds. FIG. 14 shows that the stop of oxygen supply to the patient during the time between the points a3 and a4 is performed in the interval between the point a2 and the point a1 after the point a2 (i.e., during the exhalation time).

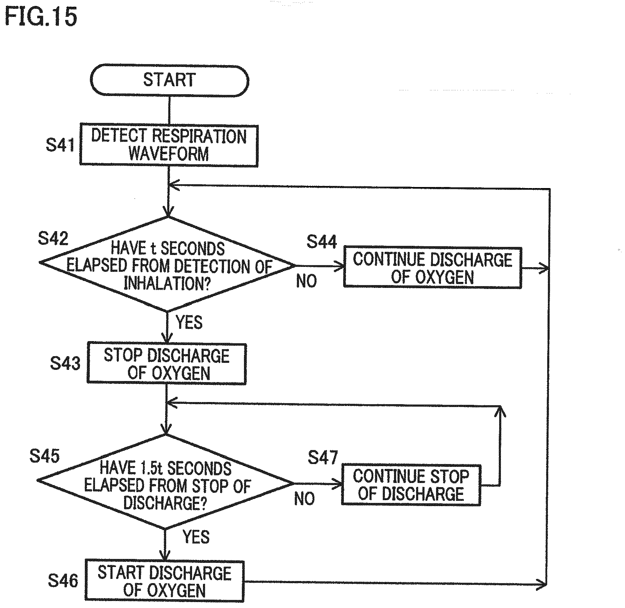

A block diagram of the oxygen concentrator 10 is identical with that of First Embodiment shown in FIG. 1. The following will describe a flow of stop and start of oxygen supply by the oxygen concentrator 10 of Sixth Embodiment with reference to FIG. 15.

After oxygen supply to a patient starts, the respiration waveform sensor 20 detects a waveform of patient's respiration (step S41). After the step S41, the control unit 23 determines whether t seconds have elapsed from the detection of the inhalation by the respiration waveform sensor 20 (step S42). When t seconds have elapsed from the detection of the inhalation by the respiration waveform sensor 20 (Yes in the step S42), the control unit 23 controls the flow amount adjustment unit 22 to stop the discharge of oxygen for 1.5t seconds in the exhalation time (step S43). When t seconds have not elapsed from the detection of the inhalation by the respiration waveform sensor 20 (No in the step S42), the control unit 23 controls the flow amount adjustment unit 22 to continue the discharge of oxygen (step S44) . After the step S44, the control unit 23 returns the process to the step S42.

After the step S43, the control unit 23 determines whether 1.5t seconds have elapsed from the stop of the discharge of oxygen (step S45). When 1.5t seconds have elapsed from the stop of the discharge of oxygen (Yes in the step S45), the control unit 23 controls the flow amount adjustment unit 22 to start the discharge of oxygen (step S46). Thereafter, the control unit 23 returns the process to the step S42. When 1.5t seconds have not elapsed from the stop of the discharge of oxygen (No in the step S45), the control unit 23 controls the flow amount adjustment unit 22 to keep the oxygen discharge stopped (step S47) . After the step S47, the control unit 23 returns the process to the step S45.

In the oxygen concentrator 10 of the present invention, oxygen supply is stopped for a predetermined time during the exhalation time. This reduces an amount of supplied oxygen, and hence the oxygen tank can be used for a longer time. To put it differently, stop of oxygen supply is triggered by the detection of exhalation by a patient. When exhalation of a patient is not detected (e.g., when the respiration waveform sensor 20 does not detect a waveform of patient's respiration), oxygen supply is continued. This allows the patent to receive oxygen even during sleep.

Thus, the embodiments of the present teaching have been described hereinabove. However, the specific structure of the present invention shall not be interpreted as to be limited to the above described embodiments. The scope of the present teaching is defined not by the above embodiment but by claims set forth below, and shall encompass the equivalents in the meaning of the claims and every modification within the scope of the claims.

While the respiration waveform sensor 20 is connected to the passage 14 in the embodiments above, the disclosure is not limited to this arrangement. The effects of the embodiments can be attained when, for example, the respiration waveform sensor 20 is directly connected to the cannula 2.

The shapes and locations of the oxygen generation unit 12 and the oxygen discharge unit 13 are not limited. The respiration waveform sensor 20 is not limited to a capacitor microphone on condition that a waveform of patient's respiration is directly detectable.

### [Reference Signs List]

2 cannula
4 contact portion
10 oxygen concentrator
12 oxygen generation unit
13 oxygen discharge unit
14 passage
20 respiration waveform sensor
21 respiration waveform storage unit
22 flow amount adjustment unit
23 control unit
26 state detection unit
27 flow amount determination unit
28 flow amount storage unit
31 fire detection unit
32 fire waveform storage unit
35 correction unit
36 correction amount determination unit
37 input unit
38 correction amount storage unit

## Claims

1. An oxygen concentrator (10) comprising:
an oxygen generation unit (12) configured to generate oxygen; an oxygen discharge unit (13) configured to discharge the oxygen generated by the oxygen generation unit (12);
a respiration waveform sensor (20) connected to at least one of a passage (14) and a cannula (2), the passage (14) connecting the oxygen generation unit (12) to the oxygen discharge unit (13), and the cannula (2) attached to the oxygen discharge unit (13) ;
a respiration waveform storage unit (21) configured to store a respiration waveform detected by the respiration waveform sensor (20);
a flow amount adjustment unit (22) provided on the passage (14) ; and
a control unit (23) configured to control the flow amount adjustment unit (22) based on the respiration waveform detected by the respiration waveform sensor (20),
**characterized in that**
the control unit (23) controls the flow amount adjustment unit (22)so that:
oxygen supply from the oxygen discharge unit (13) is continued, when the respiration waveform sensor (20) does not detect an exhalation by a patient; and
oxygen supply from the oxygen discharge unit (13) is stopped for a predetermined time during an exhalation time, when the respiration waveform sensor (20) detects an exhalation by the patient.

2. The oxygen concentrator (10) according to claim 1, further comprising:
a flow amount storage unit (28) configured to store a plurality of flow amounts corresponding to a plurality of states of a patient;
a state detection unit (26) configured to detect a state of the patient based on the respiration waveform detected by the respiration waveform sensor (20); and
a state flow amount determination unit (27) configured to determine a flow amount, corresponding to the state of the patient detected by the state detection unit (26), in the plurality of flow amounts stored in the flow amount storage unit (28),
wherein the control unit (23) controls the flow amount adjustment unit (22) so that the oxygen with the flow amount determined by the flow amount determination unit is discharged from the oxygen discharge unit (13).

3. The oxygen concentrator (10) according to claim 1 or 2, further comprising:
a fire waveform storage unit (32) configured to store a waveform of a fire; and
a fire detection unit (31) configured to detect that the respiration waveform detected by the respiration waveform sensor (20) is identical with the waveform of the fire stored in the fire waveform storage unit (32),
the control unit (23) controls the flow amount adjustment unit (22) so that the oxygen is not discharged from the oxygen discharge unit (13), when the fire detection unit (31) detects that the respiration waveform detected by the respiration waveform sensor (20) is identical with the waveform of the fire stored in the fire waveform storage unit (32).

4. The oxygen concentrator (10) according to any one of claims 1 to 3, further comprising:
an input unit (37) to which length of the cannula (2) is input; a correction amount storage unit (38) configured to store a correction amount corresponding to the length of the cannula (2) ;
a correction amount determination unit (36) configured to determine a correction amount corresponding to the length of the cannula (2) input to the input unit (37), with reference to the correction amount stored in the correction amount storage unit (38); and
a correction unit (35) configured to correct the amplitude of the respiration waveform detected by the respiration waveform sensor (20), based on the correction amount determined by the correction amount determination unit (36).

5. The oxygen concentrator (10) according to any one of claims 1 to 4, wherein,
the cannula (2) includes a contact portion (4) which is in contact with an ear of the patient when the cannula (2) is attached to a nose of the patient,
the respiration waveform sensor (20) is configured to detect heartbeat of the patient through the contact portion (4), in addition to the respiration waveform of the patient, and the respiration waveform and the heartbeat detected by the respiration waveform sensor (20) are stored in the respiration waveform storage unit (21).

## Patentansprüche

1. Sauerstoffkonzentrator (10), umfassend:
eine Sauerstofferzeugungseinheit (12), die konfiguriert ist, um Sauerstoff zu erzeugen;
eine Sauerstoffabgabeeinheit (13), die konfiguriert ist, um den von der Sauerstofferzeugungseinheit (12) erzeugten Sauerstoff abzugeben;
einen Atmungswellenform-Sensor (20), der mit mindestens einem von einem Durchgang (14) und einer Kanüle (2) verbunden ist, wobei der Durchgang (14) die Sauerstofferzeugungseinheit (12) mit der Sauerstoffabgabeeinheit (13) verbindet und die Kanüle (2) an der Sauerstoffabgabeeinheit (13) angebracht ist;
eine Atmungswellenform-Speichereinheit (21), die konfiguriert ist, um eine von dem Atmungswellenform-Sensor (20) erfasste Atmungswellenform zu speichern;
eine Durchflussmengen-Einstelleinheit (22), die am Durchgang (14) vorgesehen ist; und
eine Steuereinheit (23), die konfiguriert ist, um die Durchflussmengen-Einstelleinheit (22) auf der Grundlage der von dem Atmungswellenform-Sensor (20) erfassten Atmungswellenform zu steuern,
**dadurch gekennzeichnet, dass**
die Steuereinheit (23) die Durchflussmengen-Einstelleinheit (22) so steuert, dass:
die Sauerstoffzufuhr von der Sauerstoffabgabeeinheit (13) fortgesetzt wird, wenn der Atmungswellenform-Sensor (20) keine Ausatmung durch einen Patienten erfasst; und
die Sauerstoffzufuhr von der Sauerstoffabgabeeinheit (13) für eine vorbestimmte Zeit während einer Ausatmungszeit gestoppt wird, wenn der Atmungswellenform-Sensor (20) eine Ausatmung durch den Patienten feststellt.

2. Sauerstoffkonzentrator (10) nach Anspruch 1, ferner umfassend:
eine Durchflussmengen-Speichereinheit (28), die konfiguriert ist, um eine Vielzahl von Durchflussmengen zu speichern, die einer Vielzahl von Zuständen eines Patienten entsprechen;
eine Zustandserkennungseinheit (26), die konfiguriert ist, um einen Zustand des Patienten auf der Grundlage der von dem Atmungswellenform-Sensor (20) erfassten Atmungswellenform zu erkennen; und
eine Zustandsdurchflussmengen-Bestimmungseinheit (27), die konfiguriert ist, um eine Durchflussmenge, die dem von der Zustandserfassungseinheit (26) erfassten Zustand des Patienten entspricht, in der Vielzahl von Durchflussmengen zu bestimmen, die in der Durchflussmengen-Speichereinheit (28) gespeichert sind,
wobei die Steuereinheit (23) die Durchflussmengen-Einstelleinheit (22) so steuert, dass der Sauerstoff mit der von der Durchflussmengen-Bestimmungseinheit bestimmten Durchflussmenge aus der Sauerstoffabgabeeinheit (13) abgegeben wird.

3. Sauerstoffkonzentrator (10) nach Anspruch 1 oder 2, ferner umfassend:
eine Feuerwellenform-Speichereinheit (32), die konfiguriert ist, um eine Wellenform eines Feuers zu speichern; und
eine Feuererkennungseinheit (31), die konfiguriert ist, um zu erkennen, dass die von dem Atmungswellenform-Sensor (20) erkannte Atmungswellenform mit der Wellenform des in der Feuerwellenform-Speichereinheit (32) gespeicherten Feuers identisch ist,
die Steuereinheit (23) die Durchflussmengen-Einstelleinheit (22) so steuert, dass der Sauerstoff nicht von der Sauerstoffabgabeeinheit (13) abgegeben wird, wenn die Feuererkennungseinheit (31) erkennt, dass die von dem Atmungswellenform-Sensor (20) erfasste Atmungswellenform mit der in der Feuerwellenform-Speichereinheit (32) gespeicherten Wellenform des Feuers identisch ist.

4. Sauerstoffkonzentrator (10) nach einem der Ansprüche 1 bis 3, ferner umfassend:
eine Eingabeeinheit (37), in die die Länge der Kanüle (2) eingegeben wird;
eine Korrekturbetrags-Speichereinheit (38), die konfiguriert ist, um einen Korrekturbetrag zu speichern, der der Länge der Kanüle (2) entspricht;
eine Korrekturbetrags-Bestimmungseinheit (36), die konfiguriert ist, um einen Korrekturbetrag entsprechend der in die Eingabeeinheit (37) eingegebenen Länge der Kanüle (2) unter Bezugnahme auf den in der Korrekturbetrags-Speichereinheit (38) gespeicherten Korrekturbetrag zu bestimmen; und
eine Korrektureinheit (35), die konfiguriert ist, um die Amplitude der von dem Atmungswellenform-Sensor (20) erfassten Atmungswellenform auf der Grundlage des von der Korrekturbetrags-Bestimmungseinheit (36) bestimmten Korrekturbetrags zu korrigieren.

5. Sauerstoffkonzentrator (10) nach einem der Ansprüche 1 bis 4, wobei
die Kanüle (2) einen Kontaktabschnitt (4) aufweist, der in Kontakt mit einem Ohr des Patienten steht, wenn die Kanüle (2) an einer Nase des Patienten angebracht ist,
der Atmungswellenform-Sensor (20) konfiguriert ist, um den Herzschlag des Patienten durch den Kontaktabschnitt (4) zusätzlich zur Atmungswellenform des Patienten zu erfassen, und
die Atmungswellenform und der Herzschlag, die von dem Atmungswellenform-Sensor (20) erfasst werden, in der Atmungswellenform-Speichereinheit (21) gespeichert werden.

## Revendications

1. Concentrateur d'oxygène (10) comprenant :
une unité de génération d'oxygène (12) configurée pour générer de l'oxygène ;
une unité de décharge d'oxygène (13) configurée pour décharger l'oxygène généré par l'unité de génération d'oxygène (12) ;
un capteur de forme d'onde de respiration (20) raccordé à au moins l'un d'entre un passage (14) et une canule (2), le passage (14) raccordant l'unité de génération d'oxygène (12) à l'unité de décharge d'oxygène (13), et la canule (2) étant attachée à l'unité de décharge d'oxygène (13) ;
un capteur de stockage de forme d'onde de respiration (21) configuré pour stocker une forme d'onde de respiration détectée par le capteur de forme d'onde de respiration (20) ;
une unité d'ajustement de quantité d'écoulement (22) fournie sur le passage (14) ; et
une unité de contrôle (23) configurée pour contrôler l'unité d'ajustement de quantité d'écoulement (22) sur la base de la forme d'onde de respiration détectée par le capteur de forme d'onde de respiration (20),
**caractérisé en ce que**
l'unité de contrôle (23) contrôle l'unité d'ajustement de quantité d'écoulement (22) de telle sorte que :
l'alimentation en oxygène de l'unité de décharge d'oxygène (13) est poursuivie lorsque le capteur de forme d'onde de respiration (20) ne détecte pas une exhalation par un patient ; et
l'alimentation en oxygène de l'unité de décharge d'oxygène (13) est arrêtée pendant un temps prédéterminé durant un temps d'exhalation, lorsque le capteur de forme d'onde de respiration (20) détecte une exhalation par le patient.

2. Concentrateur d'oxygène (10) selon la revendication 1, comprenant en outre :
une unité de stockage de quantités d'écoulement (28) configurée pour stocker une pluralité de quantités d'écoulement correspondant à une pluralité d'états d'un patient ;
une unité de détection d'état (26) configurée pour détecter un état du patient sur la base de la forme d'onde de respiration détectée par le capteur de forme d'onde de respiration (20) ; et
une unité de détermination de quantité d'écoulement pour un état (27) configurée pour déterminer une quantité d'écoulement, correspondant à l'état du patient détecté par l'unité de détection d'état (26), parmi la pluralité de quantités d'écoulement stockées dans l'unité de stockage de quantités d'écoulement (28),
dans lequel l'unité de contrôle (23) contrôle l'unité d'ajustement de quantité d'écoulement (22) de telle sorte que de l'oxygène avec la quantité d'écoulement déterminée par l'unité de détermination de quantité d'écoulement est déchargé de l'unité de décharge d'oxygène (13).

3. Concentrateur d'oxygène (10) selon la revendication 1 ou 2, comprenant en outre :
une unité de stockage de forme d'onde de feu (32) configurée pour stocker une forme d'onde d'un feu ; et
une unité de détection de feu (31) configurée pour détecter que la forme d'onde de respiration détectée par le capteur de forme d'onde de respiration (20) est identique à la forme d'onde du feu stockée dans l'unité de stockage de forme d'onde de feu (32),
l'unité de contrôle (23) contrôle l'unité d'ajustement de quantité d'écoulement (22) de telle sorte que de l'oxygène n'est pas déchargé de l'unité de décharge d'oxygène (13), lorsque l'unité de détection de feu (31) détecte que la forme d'onde de respiration détectée par le capteur de forme d'onde de respiration (20) est identique à la forme d'onde du feu stockée dans l'unité de stockage de forme d'onde de feu (32).

4. Concentrateur d'oxygène (10) selon l'une quelconque des revendications 1 à 3, comprenant en outre :
une unité d'entrée (37) dans laquelle une longueur de la canule (2) est entrée ;
une unité de stockage de quantité de correction (38) configurée pour stocker une quantité de correction correspondant à la longueur de la canule (2) ;
une unité de détermination de quantité de correction (36) configurée pour déterminer une quantité de correction correspondant à la longueur de la canule (2) entrée dans l'unité d'entrée (37), en se référant à la quantité de correction stockée dans l'unité de stockage de quantité de correction (38) ; et
une unité de correction (35) configurée pour corriger l'amplitude de la forme d'onde de respiration détectée par le capteur de forme d'onde de respiration (20), sur la base de la quantité de correction déterminée par l'unité de détermination de quantité de correction (36).

5. Concentrateur d'oxygène (10) selon l'une quelconque des revendications 1 à 4, dans lequel,
la canule (2) comprend une partie de contact (4) qui est en contact avec une oreille du patient lorsque la canule (2) est attachée à un nez du patient,
le capteur de forme d'onde de respiration (20) est configuré pour détecter un battement de cœur du patient par la partie de contact (4), en plus de la forme d'onde de respiration du patient, et la forme d'onde de respiration et le battement de cœur détectés par le capteur de forme d'onde de respiration (20) sont stockés dans l'unité de stockage de forme d'onde de respiration (21).
